(19) Europäisches Patentamt
European Patent Office
Office européen des brevets

(11) Publication number: 0 562 479 A1

(12) **EUROPEAN PATENT APPLICATION**

(21) Application number: 93104527.2

(22) Date of filing: 19.03.93

(51) Int. Cl.5: C07D 401/04, C07D 409/04, A01N 43/653

(30) Priority: 27.03.92 JP 102148/92

(43) Date of publication of application: 29.09.93 Bulletin 93/39

(84) Designated Contracting States: DE FR GB

(71) Applicant: KUREHA CHEMICAL INDUSTRY CO., LTD.
9-11, Horidome-cho, 1-chome Nihonbashi
Chuo-ku Tokyo 103(JP)

(72) Inventor: Watanabe, Takeo
78-31, Hananoi, Nishiki-machi
Iwaki-shi, Fukushima(JP)
Inventor: Saishoji, Toshihide
154-1, Harada, Nishiki-machi
Iwaki-shi, Fukushima(JP)
Inventor: Shida, Takafumi
11-2, Chuo 2-chome, Nishiki-machi
Iwaki-shi, Fukushima(JP)

(74) Representative: Boeters, Hans Dietrich, Dr. et al
Boeters & Bauer Bereiteranger 15
D-81541 München (DE)

(54) **1-Phenyl-5-heterocyclic-1H-1, 2,4-triazole-3-carboxamide derivates, process for preparation of the same, and fungicidal composition comprising the same.**

(57) A derivative of 1-phenyl-5-heterocyclic-1H-1,2,4-triazole-3-carboxamide represented by the following general formula (I), a process for the manufacture thereof, and an fungicidal composition comprising the same as an active ingredient.

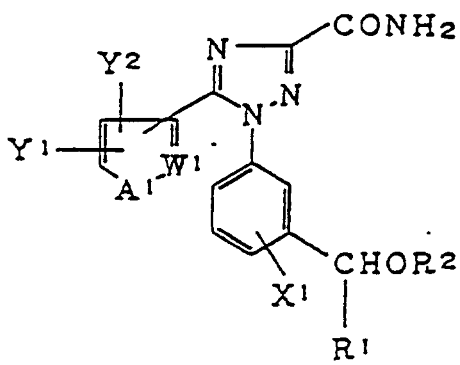

(I)

wherein $R^1$ is a $C_1$-$C_6$ alkyl group, $R^2$ is a $C_1$-$C_8$ alkyl group or a phenylmethyl group, $X^1$ represents a hydrogen atom, a $C_1$-$C_4$ alkyl group or a halogen atom, $Y^1$ is a hydrogen atom or a $C_1$-$C_4$ alkyl group, $Y^2$ is a hydrogen atom, a $C_1$-$C_4$ alkyl group, or a halogen atom, $A^1$ represents a group -N=CH-, -NH-, -N($C_1$-$C_4$ alkyl)-,-N(Ph)-, -O-, or -S-, and $W^1$ denotes a group -C($W^2$)= or -N=, wherein $W^2$ is a hydrogen atom, a $C_1$-$C_4$ alkyl group, or a halogen atom.

EP 0 562 479 A1

## BACKGROUND OF THE INVENTION

### Field of the Invention

The present invention relates to a derivative of 1-phenyl-5-heterocyclic-1H-1,2,4-triazole-3-carboxamide, to a process for manufacturing the same, and to a fungicidal composition comprising the same.

### Description of Background Art

Prior arts describing derivatives of 1,5-diphenyl-1H-1,2,4-triazole-3-carboxamide include EP-282303-A, EP-282669-A, EP-220956-A, and the like.

All derivatives of 1,5-diphenyl-1H-1,2,4-triazole-3-carboxamide described in these prior arts are herbicidal compounds, in which a substituted or unsubstituted alkoxymethyl group is bonded to the 3 position of 1-phenyl group of the triazole ring.

While said derivatives of 1,5-diphenyl-1H-1,2,4-triazole-3-carboxamide with a substituted or unsubstituted alkoxymethyl group bonded to the 3 position of 1-phenyl group of the triazole ring described in the above prior arts are useful as a herbicidal composition, the present inventors have discovered that a novel compound of which hydrogen atom of the methylene group is further substituted, as shown in the following formula (I), is useful as a fungicidal agent.

## SUMMARY OF THE INVENTION

Accordingly, an object of the present invention is to provide a novel derivative of 1-phenyl-5-heterocyclic-1H-1,2,4-triazole-3-carboxamide represented by the following formula (I),

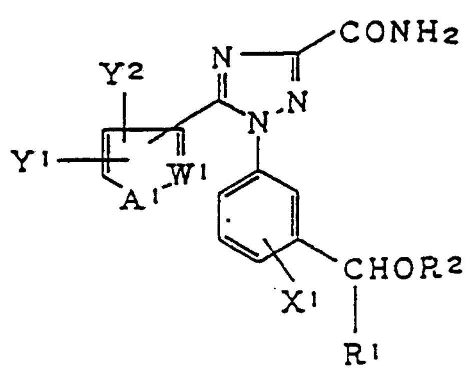

(I)

wherein $R^1$ is a $C_1$-$C_6$ alkyl group, $R^2$ is a $C_1$-$C_8$ alkyl group or a phenylmethyl group, $X^1$ represents a hydrogen atom, a $C_1$-$C_4$ alkyl group or a halogen atom, $Y^1$ is a hydrogen atom or a $C_1$-$C_4$ alkyl group, $Y^2$ is a hydrogen atom, a $C_1$-$C_4$ alkyl group, or a halogen atom, $A^1$ represents a group -N=CH-, -NH-, -N($C_1$-$C_4$ alkyl)-,-N(Ph)-, -O-, or -S-, and $W^1$ denotes a group -C($W^2$)= or -N=, wherein $W^2$ is a hydrogen atom, a $C_1$-$C_4$ alkyl group, or a halogen atom; provided that $A^1$, $W^1$ and $W^2$ form a heterocycle, $Y^1$ and $Y^2$ are bonded, and further bonded to 5-position of the 1H-1,2,4-triazole ring at the heterocycle bonding position.

Another object of the present invention is to provide a process for manufacturing said novel carboxamide derivative (I).

Still another object of the present invention is to provide a fungicidal composition, especially a fungicidal composition used for agriculture and horticulture, comprising said novel carboxamide derivative as an active ingredient.

Specifically, the present invention comprises the following features.

A first invention relates to a derivative of 1-phenyl-5-heterocyclic-1H-1,2,4-triazole-3-carboxamide (I),

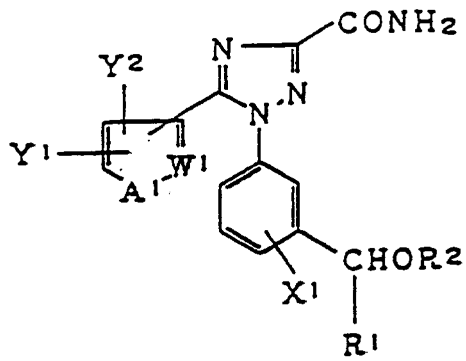

(I )

wherein $R^1$, $R^2$, $X^1$, $Y^1$, $Y^2$, $A^1$, $W^1$, and $Y^2$ have the same meanings as defined above.

A second invention relates to a process for manufacturing said derivative of 1-phenyl-5-heterocyclic-1H-1,2,4-triazole-3-carboxamide (I), which comprises reacting oxazoledione hydrazone derivative (VI) and ammonia to produce glycinamide derivative (VII) and subjecting the glycinamide derivative (VII) to a cyclocondensation reaction according to the following reaction formula (I),

**Reaction (I)**

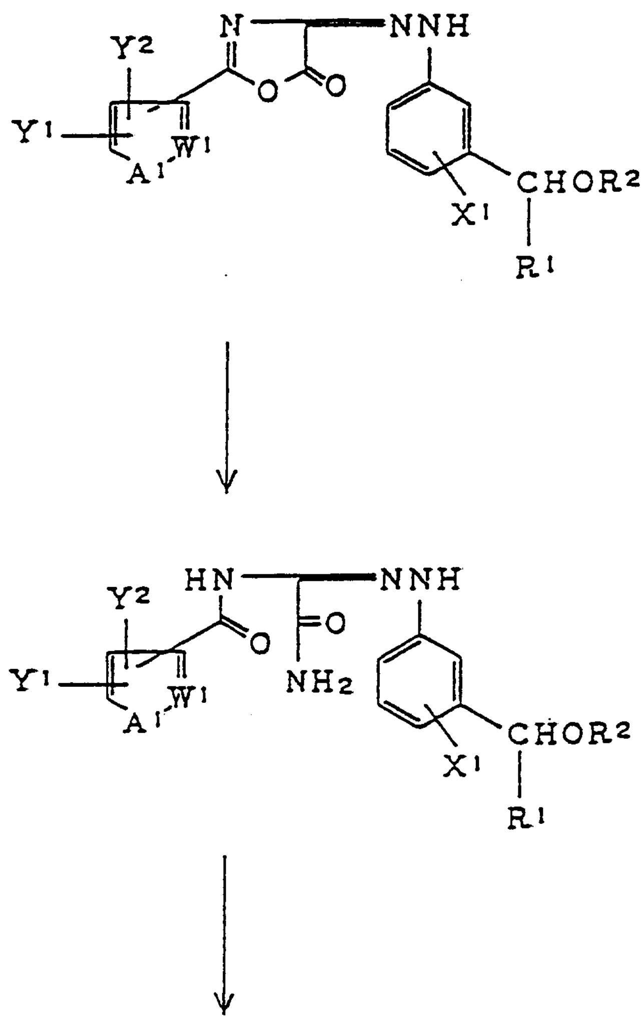

(VI)

(VII )

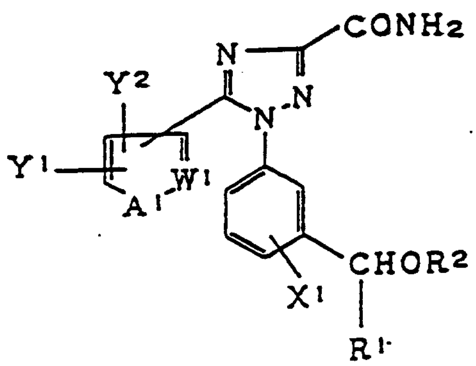

(I )

wherein $R^1$, $R^2$, $X^1$, $Y^1$, $Y^2$, $A^1$, $W^1$, and $Y^2$ have the same meanings as defined above.

A third invention relates to a process for manufacturing said derivative of 1-phenyl-5-hetrocyclic-1H-1,2,4-triazole-3-carboxamide, which comprises reacting oxamide derivative (X) and aldehyde derivative (XI) to produce dihydrotriazole carboxamide (XII) and oxidizing the dihydrotriazole carboxamide (XII) according to the following reaction formula (II),

**Reaction (II)**

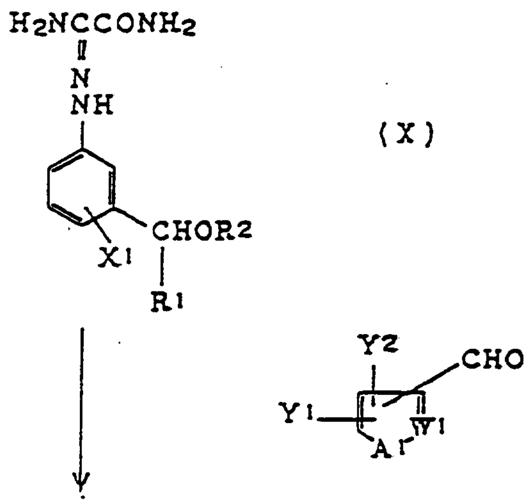

(X)

(XI)

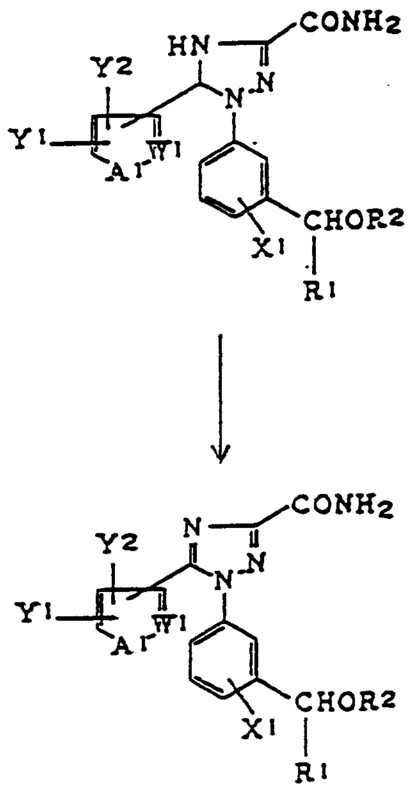

(XII)

(I)

wherein $R^1$, $R^2$, $X^1$, $Y^1$, $Y^2$, $A^1$, $W^1$, and $Y^2$ have the same meanings as defined above.

A fourth invention relates to a fungicidal composition comprising said dervative of 1-phenyl-5-heterocyclic-1H-1,2,4-triazole-3-carboxamide (I) as an active ingredient.

Other objects, features and advantages of the invention will hereinafter become more readily apparent from the following description.

## DETAILED DESCRIPTION OF THE INVENTION AND PREFERRED EMBODIMENTS

Given as specific examples of derivatives of 1-phenyl-5-heterocyclic-1H-1,2,4-triazole-3-carboxamide (I) are compounds in the following Table 1. Physicochemical characteristics of the compounds of Table 1 are listed in Table 2.

Since compound (I) has a triazole ring, it may be used in forms such as inorganic acid salts, organic acid salts or metal complexes. The present invention includes such salts and complexes.

In addition, there are optical isomers for compound (I) due to the substituent at a 3 position of 1-phenyl group. All of such optical isomers as well as mixtures of the isomers at any ratio are also included in the present invention.

TABLE 1

| Compound | Substituents $R^1$ / $R^2$ / $X^1$ | $A^1$ / $W^1$ / $W^2$ | 5-Heterocycle bonding position / $Y^1$ / $Y^2$ |
|---|---|---|---|
| 1 | $CH_3CH_2$<br>$CH_3CH_2CH_2CH_2$<br>6-$CH_3$ | -N=CH-<br>-C($W^2$)=<br>H | 2<br>6-$CH_3$<br>H |
| 2 | $CH_3$<br>$CH_3CH_2CH_2CH_2$<br>6-Cl | -N=CH-<br>-C($W^2$)=<br>H | 2<br>6-$CH_3$<br>H |

TABLE 1 (Continued)

| Compound | Substituents | | |
|---|---|---|---|
| | $R^1$<br>$R^2$<br>$X^1$ | $A^1$<br>$W^1$<br>$W^2$ | 5-Heterocycle bonging posilion<br>$Y^1$<br>$Y^2$ |
| 3 | $CH_3(CH_3)CH$<br>$CH_3CH_2CH_2CH_2$<br>6-$CH_3$ | -N=CH-<br>-C($W^2$)=<br>H | 2<br>6-$CH_3$<br>H |
| 4 | $CH_3(CH_3)CH$<br>$CH_3CH_2CH_2CH_2$<br>H | -N=CH-<br>-C($W^2$)=<br>H | 2<br>H<br>H |
| 5 | $CH_3(CH_3)CH$<br>$CH_3CH_2CH_2CH_2$<br>H | -N=CH-<br>-C($W^2$)=<br>H | 4<br>H<br>H |
| 6 | $CH_3(CH_3)CH$<br>$CH_3CH_2CH_2CH_2$<br>H | -N=CH-<br>-C($W^2$)=<br>H | 3<br>H<br>H |
| 7 | $CH_3(CH_3)CH$<br>$CH_3CH_2CH_2CH_2$<br>H | -N=CH-<br>-C($W^2$)=<br>H | 2<br>6-$CH_3$<br>H |
| 8 | $CH_3(CH_3)CH$<br>$CH_3CH_2CH_2CH_2$<br>H | -N($CH_3$)-<br>-C($W^2$)=<br>H | 2<br>H<br>H |
| 9 | $CH_3(CH_3)CH$<br>$CH_3CH_2CH_2CH_2$<br>H | -N(Ph)-<br>-C($W^2$)=<br>$CH_3$ | 3<br>2-$CH_3$<br>H |
| 10 | $CH_3(CH_3)CH$<br>$CH_3CH_2CH_2CH_2$<br>H | -O-<br>-C($W^2$)=<br>H | 3<br>H<br>H |
| 11 | $CH_3(CH_3)CH$<br>$CH_3CH_2CH_2CH_2$<br>H | -O-<br>-C($W^2$)=<br>H | 2<br>H<br>H |
| 12 | $CH_3(CH_3)CH$<br>$CH_3CH_2CH_2CH_2$<br>6-$CH_3$ | -O-<br>-N= | 4<br>3-$CH_3$<br>5-$CH_3$ |

TABLE 1 (Continued)

| Compound | Substituents $R^1$ $R^2$ $X^1$ | $A^1$ $W^1$ $W^2$ | 5-Heterocycle bonging posilion $Y^1$ $Y^2$ |
|---|---|---|---|
| 13 | $CH_3CH_2$<br>$CH_3CH_2CH_2CH_2$<br>6-$CH_3$ | -S-<br>-C($W^2$)=<br>H | 2<br>3-$CH_3$<br>H |
| 14 | $CH_3$<br>$CH_3CH_2CH_2CH_2$<br>6-Cl | -S-<br>-C($W^2$)=<br>H | 2<br>3-$CH_3$<br>H |
| 15 | $CH_3(CH_3)CH$<br>$CH_3CH_2CH_2CH_2$<br>H | -S-<br>-C($W^2$)=<br>H | 2<br>H<br>H |
| 16 | $CH_3(CH_3)CH$<br>$CH_3CH_2CH_2CH_2$<br>6-$CH_3$ | -S-<br>-C($W^2$)=<br>H | 2<br>3-$CH_3$<br>H |
| 17 | $CH_3(CH_3)CH$<br>$CH_3CH_2CH_2CH_2$<br>H | -S-<br>-C($W^2$)=<br>$CH_3$ | 2<br>H<br>H |
| 18 | $CH_3(CH_3)CH$<br>$CH_3CH_2CH_2CH_2$<br>H | -S-<br>-C($W^2$)=<br>H | 3<br>H<br>H |
| 19 | $CH_3(CH_3)CH$<br>$CH_3CH_2CH_2CH_2$<br>H | -S-<br>-C($W^2$)=<br>H | 2<br>3-$CH_3$<br>H |
| 20 | $CH_3(CH_3)CH$<br>$PhCH_2$<br>H | -S-<br>-C($W^2$)=<br>Br | 2<br>H<br>H |
| 21 | $CH_3(CH_3)CH$<br>$PhCH_2$<br>H | -S-<br>-C($W^2$)=<br>H | 2<br>3-$CH_3$<br>H |

TABLE 2

| Compound No. | Physicochemical Characteristics |
|---|---|
| 1 | Appearence: Colorless crystal<br>m.p. (°C) : 165-166<br>IR ($cm^{-1}$) : 3470, 3230, 1700, 1670 |
| 2 | Appearence: Colorless crystal<br>m.p. (°C) : 177-178<br>IR ($cm^{-1}$) : 3480, 3230, 1710, 1670 |
| 3 | Appearence: Colorless crystal<br>m.p. (°C) : 145-146<br>IR ($cm^{-1}$) : 3450, 3250, 1650 |
| 4 | Appearence: Light brown crystal<br>m.p. (°C) : 127-129<br>IR ($cm^{-1}$) : 3425, 3275, 1675 |
| 5 | Appearence: Light brown crystal<br>m.p. (°C) : 78-80<br>IR ($cm^{-1}$) : 3430, 3290, 1690 |
| 6 | Appearence: Light brown crystal<br>m.p. (°C) : 130-132<br>IR ($cm^{-1}$) : 3400, 3300, 1685 |
| 7 | Appearence: Colorless crystal<br>m.p. (°C) : 133-135<br>IR ($cm^{-1}$) : 3480, 3230, 1710, 1670 |
| 8 | Appearence: Light brown crystal<br>m.p. (°C) : 118-120<br>IR ($cm^{-1}$) : 3425, 3280, 1690 |
| 9 | Appearence: Light brown liquid<br>m.p. (°C) :<br>IR ($cm^{-1}$) : 3455, 3290, 1685 |
| 10 | Appearence: Colorless crystal<br>m.p. (°C) : 80-82<br>IR ($cm^{-1}$) : 3350, 3140, 1685 |
| 11 | Appearence: Colorless crystal<br>m.p. (°C) : 146-148<br>IR ($cm^{-1}$) : 3420, 3300, 1670 |

TABLE 2 (Continued)

| Compound No. | Physicochemical Characteristics |
|---|---|
| 12 | Appearence: Colorless crystal<br>m.p. (°C) : 127-128<br>IR ($cm^{-1}$) : 3320, 3150, 1670 |
| 13 | Appearence: Light brown liquid<br>m.p. (°C) :<br>IR ($cm^{-1}$) : 3450, 3150, 1680 |
| 14 | Appearence: Light brown liquid<br>m.p. (°C) :<br>IR ($cm^{-1}$) : 3450, 3160, 1680 |
| 15 | Appearence: Gray crystal<br>m.p. (°C) : 122-124<br>IR ($cm^{-1}$) : 3430, 3280, 1685 |
| 16 | Appearence: Light brown liquid<br>m.p. (°C) :<br>IR ($cm^{-1}$) : 3450, 3170, 1680 |
| 17 | Appearence: Gray crystal<br>m.p. (°C) : 151-153<br>IR ($cm^{-1}$) : 3465, 3260, 1710, 1670 |
| 18 | Appearence: Gray crystal<br>m.p. (°C) : 95-97<br>IR ($cm^{-1}$) : 3470, 3270, 1670 |
| 19 | Appearence: Colorless crystal<br>m.p. (°C) : 71-73<br>IR ($cm^{-1}$) : 3440, 3280, 1670 |
| 20 | Appearence: Colorless crystal<br>m.p. (°C) : 131-133<br>IR ($cm^{-1}$) : 3450, 3350, 1670 |
| 21 | Appearence: Light yellow crystal<br>m.p. (°C) : 106-108<br>IR ($cm^{-1}$) : 3460, 3310, 1690 |

The following processes (A) or (B) can be employed for manufacturing derivatives of 1-phenyl-5-heterocyclic-1H-1,2,4-triazole-3-carboxamide.

⟨Process (A)⟩

According to the above reaction formula (I), oxazoledione hydrazone derivative (VI) and ammonia are reacted in an organic solvent to produce glycinamide derivative (VII). The glycinamide derivative (VII) is converted into compound (I) by a cyclocondensation reaction in an organic solvent in the presence of an acid catalyst.

〈Process (B)〉

According to the above reaction formula (II), oxamide derivative (X) and aldehyde derivative (XI) are reacted in an organic solvent to produce dihydrotriazole carboxamide (XII). The dihydrotriazole carboxamide (XII) is oxidized into compound (I).

Processes for producing the oxazoledione hydrazone derivative (VI) used in Process (A) are discussed in detail.

〈Diazotization and diazo coupling reaction〉

According to the reaction (III) shown below, aniline derivative (II) is converted into diazonium salt derivative (III) by adding dropwise an aqueous solution of nitrite such as sodium nitrite to a mixture of aniline derivative (II), an inorganic acid, e.g., hydrochloric acid, sulfuric acid, etc., and an organic acid, e.g., acetic acid, propionic acid, etc., while cooling and with stirring. The diazonium salt derivative (III) thus obtained is reacted with a glycine derivative (IV), acetic anhydride, and a basic compound to produce oxazoledione hydrazone derivative (VI). Given as basic compounds used here are alkali metal salts of weak acid (e.g., sodium acetate), alkaline earth metal salts of weak acid (e.g., calcium acetate), alkaline earth metal oxides (e.g., calcium oxide, magnesium oxide), and tertiary amines (e.g., pyridine derivatives, triethylamine, tripropylamine). Alternatively, glycine derivative (IV) may be used for the diazo coupling reaction after converting it into oxazolone derivative (V) by heating together with acetic anhydride.

**Reaction (III)**

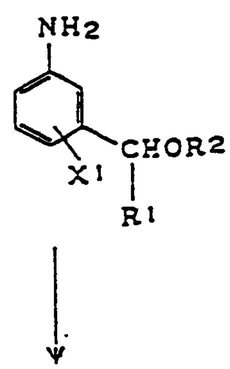

(II)

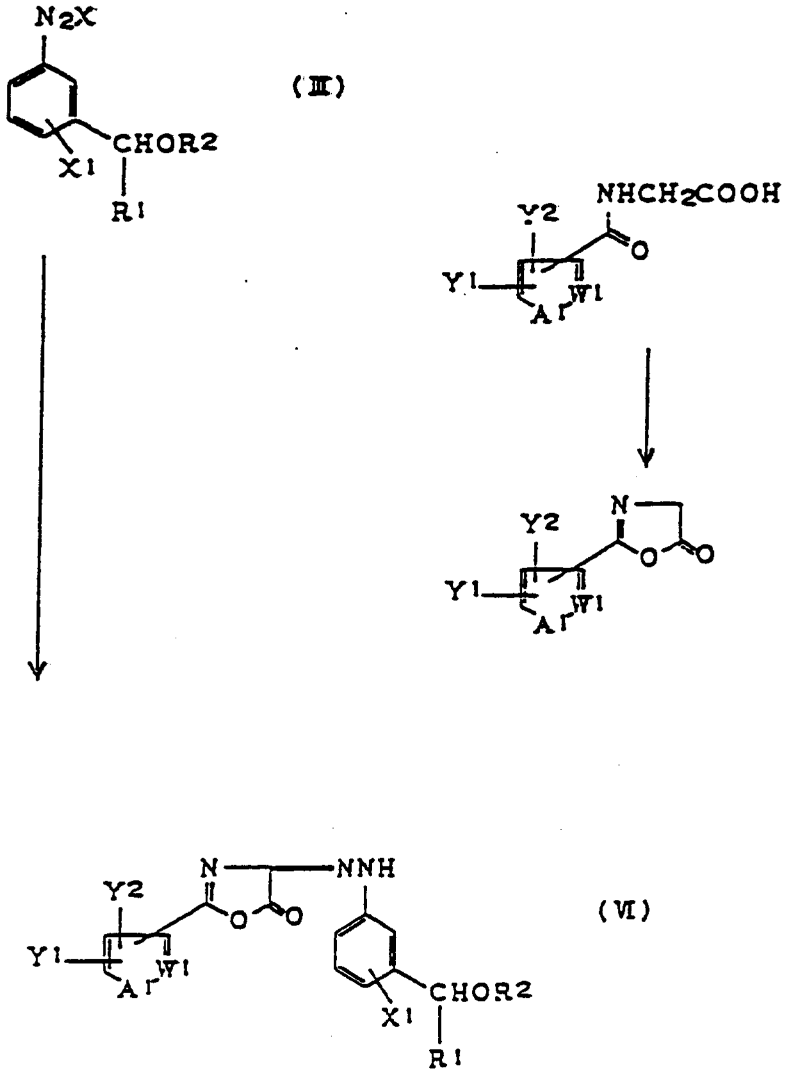

(III)

(IV)

(V)

(VI)

wherein $R^1$, $R^2$, $X^1$, $Y^1$, $Y^2$, $A^1$, $W^1$, and $Y^2$ have the same meanings as defined above (provided that $A^1$, $W^1$ and $W^2$ form a heterocycle), and $Y^1$ and $Y^2$ are bonded, said heterocycle being a part of the structure, and X denotes an anion such as chlorine ion, sulfate ion, boron tetrafluoride ion, or the like.

⟨Acylation reaction, nitration reaction, and reduction of carbonyl group⟩

Aniline derivative (II) can be obtained via nitrobenzyl alcohol derivative (XVII) according to the reaction (IV) shown below. Benzene derivative (XIII) is acylated by acyl halide (XIV) or carboxylic acid anhydride in the presence of Lewis acid, e.g., $AlCl_3$, $AlBr_3$, $ZnCl_2$, or $BF_3$, preferably $AlCl_3$, to produce ketone derivative (XV). In this reaction, an excessive amount of the reaction substrate, nitrobenzene, carbon disulfide, or the like, can be used as a reaction solvent. The ketone derivative (XV) is converted into nitrophenyl ketone derivative (XVI) by nitration using nitric acid in a mixture of nitric acid and sulfuric acid or in acetic acid solvent. Nitrobenzyl alcohol derivative (XVII) can be produced by reducing the carbonyl group of the nitrophenyl ketone derivative (XVI) thus obtained. The use of sodium borohydride as a reducing agent and an alcohol, e.g., ethyl alcohol, is preferred for the reducing reaction.

**Reaction (IV)**

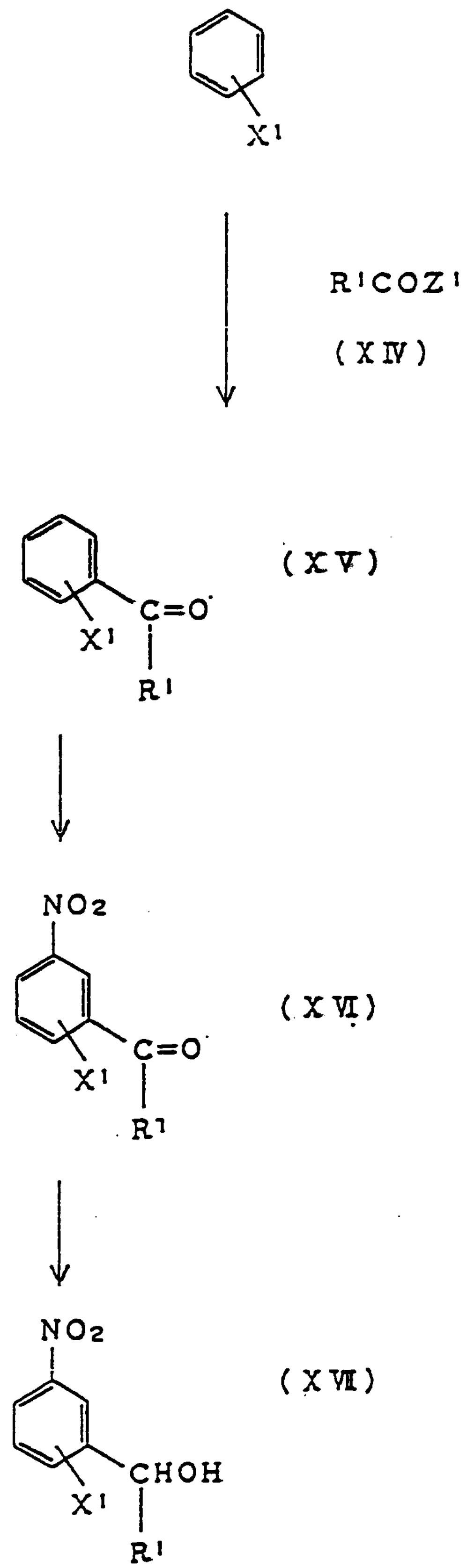


(XIII)

(XV)

(XVI)

(XVII)

wherein $R^1$ and $X^1$ have the same meanings as defined above, and $Z^1$ represents a halogen atom or a group $R^1COO$, preferably Cl or Br.

⟨Etherification reaction and nitro group reducing reaction⟩

According to the reaction (V) shown below, nitrobenzyl alcohol derivative (XVII) is alkylated with alkylating agent, such as alkyl halide (XVIII), alkyl sulfonate, or the like, to produce nitrobenzyl ether derivative (XIX). An acid-binding agent used in this reaction is a basic compound such as, for example, hydroxide or carbonate of alkali metal and hydroxide, oxide or carbonate of alkaline earth metal, or the like, preferably potassium hydroxide, sodium hydride, potassium carbonate, and the like.

The reaction is carried out preferably in the presence of a solvent, such as an aprotic polar solvent, e.g., dimethylformamide, dimethylacetamide, N-methylpyrrolidone, dimethylsulfoxide, tetrahydrofuran, sulfolane, acetonitrile, etc. The reaction temperature is usually -10 to 100 °C, and the reaction time is 0.5 to 25 hours, and preferably 1 to 10 hours. The nitro group of the nitrobenzyl ether derivative (XIX) thus obtained is reduced to produce aniline derivative (II). Bechamp reduction agent, e.g., hydrochloric acid and metallic iron; stannous chloride; catalytic hydrogenation agent, e.g., hydrogen molecule catalyzed by platinum/activated charcoal, palladium/activated charcoal, etc.; hydrazine hydrate catalyzed by activated charcoal and ferric chloride; or the like can be used in the reduction. The solvent used differs depending on the reduction method employed: an organic acid, e.g., acetic acid, propionic acid, etc., can be used for the Bechamp reduction; and an alcohol, e.g., ethyl alcohol, isopropyl alcohol, etc., an aromatic hydrocarbon, e.g., benzene, toluene, etc., or the like can be used for the catalytic reduction. The reaction temperature is usually -10 to 100 °C, and the reaction time is 0.5 to 24 hours, and preferably 1 to 10 hours.

**Reaction (V)**

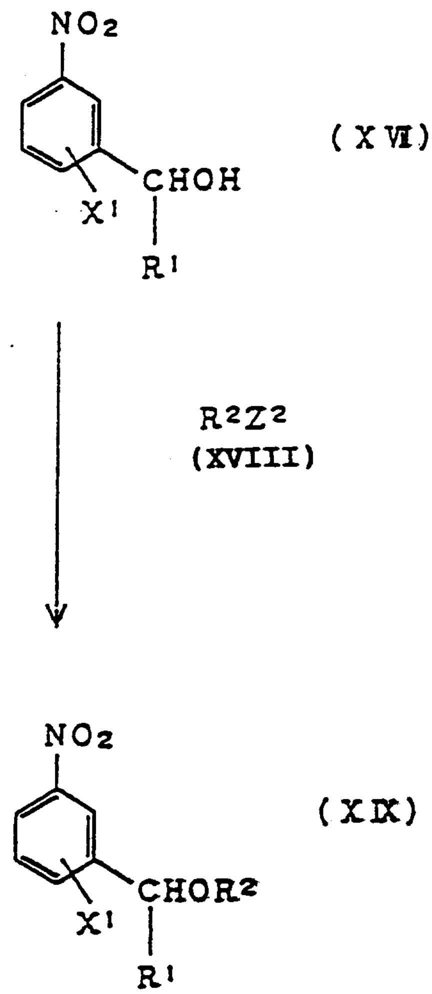

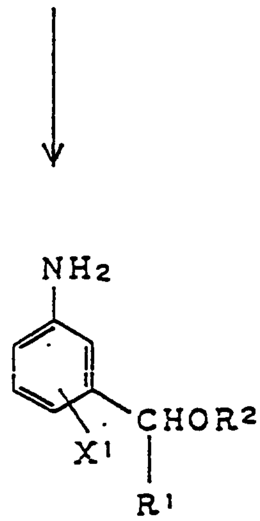

( II )

wherein $R^1$, $R^2$, and $X^1$ are the same as defined above, and $Z^2$ represents a halogen atom or a group $QSO_2O$, wherein Q is a $C_1$-$C_4$ alkyl group, substituted or unsubstituted phenyl group, preferably methyl group, phenyl group, or p-methylphenyl group.

Process (B) is now discussed in detail.

〈Diazotization and Japp-Klingemann reaction〉

Oxamide derivative (X) can be prepared as follows. According to the reaction (VI) shown below, aniline derivative (II) is converted into diazonium salt derivative (III) by adding dropwise an aqueous solution of nitrite such as sodium nitrite into a mixture of the aniline derivative (II) and an inorganic acid (e.g., hydrochloric acid, sulfuric acid), and a lower alcohol (e.g., methyl alcohol), while cooling and stirring. The diazonium salt derivative (III) is then reacted with 2-chloroacetoacetate derivative (VIII) and a basic compound, preferably in a lower alcohol such as methanol or the like, to obtain oxalyl chloride derivative (IX).

Given as basic compounds used here are alkali metal salts of weak acid (e.g., sodium acetate), alkaline earth metal salts of weak acid (e.g., calcium acetate), alkaline earth metal oxides (e.g., calcium oxide, magnesium oxide), tertiary amines (e.g., pyridine derivatives, triethylamine, tripropylamine).

The oxalyl chloride derivative and ammonia are reacted in a lower alcohol such as methanol or the like at -20 to 50 °C for 10 to 30 hours to obtain oxamide derivative (X).

**Reaction (VI)**

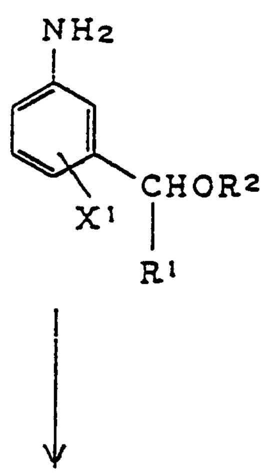

( II )

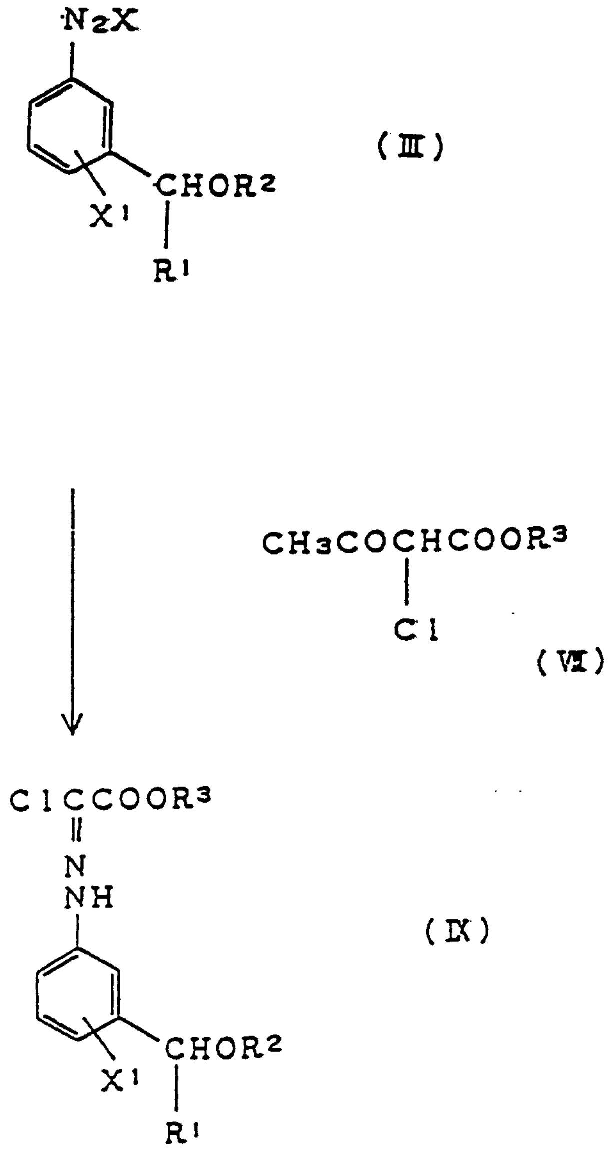
(III)

(VIII)

(IX)

wherein $R^1$, $R^2$, and $X^1$ are the same as defined above, and $R^3$ represents a $C_1$-$C_4$ alkyl group.

〈Cyclocondensation reaction and auto-oxidation reaction〉

As shown in the reaction formula (II), oxamide derivative (X) and aldehyde derivative (XI) are reacted in a lower fatty acid such as acetic acid, propionic acid, or the like to obtain dihydrotriazole carboxamide (XII), followed by auto-oxidation of the dihydrotriazole carboxamide. A cyclocondensation reaction takes place along with and the auto-oxidation reaction at a temperature of 10 to 40 °C and a reaction time for 10 to 30 hours to produce a derivative of 1-phenyl-5-heterocyclic-1H-1,2,4-triazole-3-carboxamide (I).

When the derivative of 1-phenyl-5-heterocyclic-1H-1,2,4-triazole-3-carboxamide (I) manufactured by Process (A) or Process (B) is used as a fungicidal agent, the compound is usually made into a formulation, together with adjuvants, in the form of powder, wettable or water-dispersible powder, granule, emulsion concentrate, or the like, although the compound may be used as is.

Such a formulation may contain one or more compounds of the present invention in an amount of 0.1 to 95%, preferably 0.5 to 90%, and more preferably 2 to 70% by weight.

Examples of carriers, diluents, surfactants, and the like which can be used as adjuvants in the composition of the present invention are as follows. Talc, kaolin, bentonite, diatomaceous earth, white carbon, clay, and the like, can be used as solid carriers; and water, xylene, toluene, chlorobenzene, cyclohexane, cyclohexanone, dimethylsulfoxide, dimethylformamide, alcohols, and the like are used as diluents. Different types of surfactants may be used depending on the effects intended; e.g., polyoxyethylene alkylaryl ether, polyoxyethylene sorbitan monolaurate, etc., as emulsifier; lignin sulfonate, dibutylnaphthalene sulfonate, etc., as dispersant; and alkyl sulfonate, alkylphenyl sulfonate, etc., as wetting agents.

Depending on the types such formulations are used as are or after having been diluted to a prescribed concentration with a diluent such as water.

When the formulation is used after dilution, its concentration is preferably in the range of 0.001 to 1.0%.

The compound of the present invention may be applied to fields, orchards, greenhouses, etc., in an amount of 20 to 5,000 g, preferably 50 to 1,000 g, per 1 hector.

The concentrations and amounts of use may be varied depending on types of the formulation, the time at which it is applied, the manner and the site in which it is used, and the plant to which it is applied. Thus, the amount and concentration may be increased or decreased without regard to the above specified range.

In addition, the compound of the present invention can be used in combination with other active ingredients such as, for example, fungicides, bactericides, insecticides, acaricides, or herbicides.

Other features of the invention will become apparent in the course of the following description of the exemplary embodiments which are given for illustration of the invention and are not intended to be limiting thereof.

## EXAMPLES

### Synthetic Example 1

Synthesis of
1-[3-[(1-butoxy-2-methyl)propyl]phenyl]-5-(6-methylpyridin-2-yl)-1H-1,2,4-triazole-3-carboxamide: Compound 7 in Table 1, $R^1 = CH_3(CH_3)CH$, $R^2 = CH_3CH_2CH_2CH_2$, $X^1 = H$, $A^1$ = -N = CH-, $W^1$ = -C($W^2$) = , $W^2 = H$, heterocyclic bonding position = 2, $Y^1$ = 6-$CH_3$, $Y^1 = H$, in formula (I)

⟨Synthesis by Process (B)⟩

Diazotization Reaction

50 ml of methanol, 22.1 g (99.8 mmol) of 3-[(1-butoxy-2-methyl)propyl]aniline [$R^1 = CH_3(CH_3)CH$, $R^2 = CH_3CH_2CH_2CH_2$, $X^1 = H$ in formula (II)], and 23.5 ml (264.8 mmol) of 35% hydrochloric acid were charged into a 200 ml conical flask and cooled on an ice water bath. To the mixture was added dropwise an aqueous solution of 7.3 g (105.8 mmol) of $NaNO_2$ dissolved in 10 ml of water to produce 3-((1-butoxy-2-methyl)propyl]benzene diazonium chloride [$R^1 = CH_3(CH_3)CH$, $R^2 = CH_3CH_2CH_2CH_2$, $X^1 = H$ in formula (III)].

Japp-Klingemann Reaction

15 g (99.6 mmol) of methyl chloroacetoacetate [$R^3 = CH_3$ in formula (VIII)], 50 ml of methanol, and 20 g (243.8 mmol) of sodium acetate were measured and charged into a 500 ml conical flask, and cooled on an ice water bath. After the dropwise addition of the diazonium chloride solution prepared above, the mixture was reacted for 1 hour. The ice water bath was dismantled and the reaction was continued for a further 5 hours at room temperature. The precipitated product was collected by filtration, washed with water, and dried in air to obtain 32.3 g (94.8 mmol) of methyl chloro[[3-[(1-butoxy-2-methyl)propyl]phenyl]-hydrazono] acetate [$R^1 = CH_3(CH_3)CH$, $R^2 = CH_3CH_2CH_2CH_2$, $X^1 = H$, $R^3 = CH_3$ in formula (IX)] at an yield of 95.2%.

Ammonolysis

17 g (49.9 mmol) of methyl chloro[[3-[(1-butoxy-2-methyl)propyl]phenyl]hydrazono] acetate [$R^1 = CH_3$-($CH_3$)CH, $R^2 = CH_3CH_2CH_2CH_2$, $X^1 = H$, $R^3 = CH_3$ in formula (IX)] was weighed and charged into a 300 ml eggplant type flask. After the addition of 100 ml of methanol containing 13% of ammonia, the flask was capped and allowed to stand still at room temperature for 24 hours. The reaction liquid thus obtained was concentrated and shaken thoroughly with the addition of ethyl acetate, washed with water, and dried to quantitatively obtain oxamide [3-[(1-butoxy-2-methyl)propyl]phenyl]hydrazone [$R^1 = CH_3(CH_3)CH$, $R^2 = CH_3CH_2CH_2CH_2$, $X^1 = H$, in formula (X)].

Cyclocondensation Reaction and Auto-oxidation Reaction

3.0 g (10.3 mmol) of oxamide [3-[(1-butoxy-2-methyl)propyl]phenyl]hydrazone was weighed and charged into a 50 ml eggplant type flask, followed by the addition of 30 ml of acetic acid to dissolve. To the solution was added 1.25 g (10.3 mmol) of 6-methyl-2-pyridine carboxyaldehyde [$A^1$ = -N = CH-, $W^1$ = -C($W^2$)- = , $W^2$ = H, $Y^1$ = 6-$CH_3$, $Y^2$ = H, CHO group is bonded to 2 position, in formula (XI)] and the mixture was allowed to stand at room temperature overnight.

The resultant reaction mixture was concentrated, thoroughly shaken with the addition of ethyl acetate, washed with water, dried, and concentrated to produce a crude product. This crude product was purified by column chromatography using a 10:1 (v/v) mixture of chloroform and acetone as a solvent to obtain 2.7 g of the title compound, 1-[3-[(1-butoxy-2-methyl)propyl]phenyl]-5-(6-methylpyridin-2-yl)-1H-1,2,4-triazole-3-carboxamide [Compound 7, $R^1 = CH_3(CH_3)CH$, $R^2 = CH_3CH_2CH_2CH_2$, $X^1$ = H, $A^1$ = -N = CH-, $W^1$ = -C($W^2$) = , $W^2$ = H, heterocyclic bonding position = 2, $Y^1$ = 6-$CH_3$, $Y^1$ = H, in formula (I)], at an yield of 67.0%.

The physicochemical characteristics of Compound 7 are shown in Table 2.

**Synthetic Example 2**

Synthesis of
1-[3-[(1-butoxy-2-methyl)propyl]phenyl]-5-(3-methylthiophen-2-yl)-1H-1,2,4-triazole-3-carboxamide: Compound 16, $R^1 = CH_3(CH_3)CH$, $R^2 = CH_3CH_2CH_2CH_2$, $X^1$ = H, $A^1$ = S, $W^1$ = -C($W^2$) = , $W^2$ = H, heterocyclic bonding position = 2, $Y^1$ = 3-$CH_3$, $Y^2$ = H in formula (I)

3.0 g (9.8 mmol) of oxamide [3-[(1-butoxy-2-methyl)propyl]phenyl]hydrazone [$R^1 = CH_3(CH_3)CH$, $R^2 = CH_3CH_2CH_2CH_2$, $X^1$ = H in formula (X)] was weighed and charged into a 50 ml conical flask, followed by the addition of 30 ml of acetic acid to dissolve. To the solution was added 1.3 g (10.3 mmol) of 3-methyl-2-thiophene carboxaldehyde [$A^1$ = -N = CH-, $W^1$ = -C($W^2$) = , $W^2$ = H, $Y^1$ = 3-$CH_3$, $Y^2$ = H, CHO group is bonded to a 2 position in formula (XI)] and the mixture was allowed to stand at room temperature overnight.

The resultant reaction mixture was concentrated, thoroughly shaken with the addition of ethyl acetate, washed with water, dried, and concentrated to produce a crude product. This crude product was purified by column chromatography using a 10:1 (v/v) mixture of chloroform and acetone as a solvent to obtain 3.0 g of the title compound, 1-[3-[(1-butoxy-2-methyl)propyl]phenyl]-5-(3-methylthiophen-2-yl)-1H-1,2,4-triazole-3-carboxamide at an yield of 75.0%.

The physicochemical characteristics of Compound 16 are shown in Table 2.

Synthetic Example 3 (Synthesis of an intermediate compound)

Synthesis of 3-(1-butoxyethyl)aniline [$R^1 = CH_3$, $R^2 = CH_3CH_2CH_2CH_2$, $X^1$ = H in formula (II)]

a) Synthesis of α-methyl-3-nitrobenzene methanol [$R^1 = CH_3$, $X^1$ = H in formula (XVI)]

33 g (199.8 mmol) of 1-(3-nitrophenyl)ethanone [$R^1 = CH_3$, $X^1$ = H in formula (XV)] was weighed and charged into a 500 ml eggplant type flask, followed by the addition of 120 ml of ethanol. After cooling on an ice water bath, 4.2 g (111 mmol) of $NaBH_4$ was added in portions and the mixture was reacted for 30 minutes under ice cooling and for 2 hours at room temperature. The resulting reaction liquid was concentrated by evaporator and the residue was washed with water to quantitatively obtain light brown oil of α-methyl-3-nitrobenzene methanol.

b) Synthesis of 1-(1-butoxyethyl)-3-nitrobenzene [$R^1 = CH_3$, $R^2 = CH_3CH_2CH_2CH_2$, $X^1$ = H in formula (XIX)]

30 ml of n-hexane was charged into a 300 ml reaction flask, followed by the addition of 4.4 g (110 mmol) of 60% oily NaH. The mixture was thoroughly shaken to remove n-hexane by decantation. 30 ml of n-hexane was added to repeat the above procedure to remove oily component of NaH. 100 ml of DMF was added under ice cooling. To this was added 16.7 g (99.9 mmol) of α-methyl-3-nitrobenzene methanol [$R^1 = CH_3$, $X^1$ = H in formula (XVII)] in portions to convert it into Na-oxide. 16.4 g (119.7 mmol) of 1-bromo-n-butane [$R^2 = CH_3CH_2CH_2CH_2$, $Z^2$ = Br in formula (XVIII)] was added dropwise and reacted for 30 minutes under ice cooling and for 2 hours at room temperature. The resulting reaction mixture was poured into ice water and extracted with ethyl acetate to obtain 20.5 g of a crude product. This crude product was purified by column chromatography using a 5:1 mixture of n-hexane and ethyl acetate as a solvent to obtain 17.6 g

(78.8 mmol) of light brown oily product of 1-(1-butoxyethyl)-3-nitrobenzene at an yield of 78.9%.

c) Synthesis of 3-(1-butoxyethyl)aniline [$R^1 = CH_3$, $R^2 = CH_3CH_2CH_2CH_2$, $X^1 = H$ in formula (II)]

11.7 g (52.4 mmol) of 1-(1-butoxyethyl)-3-nitrobenzene, 60 ml of ethanol, 1.2 g of activated charcoal, 0.3 g of $FeCl_3 \cdot 6H_2O$, and 1 ml of hydrazine hydrate were charged into a 300 ml reaction flask. After refluxing for 15 minutes, 12 ml of hydrazine hydrate was added dropwise over 30 minutes, followed by refluxing for a further 3 hours. The reaction liquid was aspirated through a filter paper to remove activated charcoal, and the filtrate was concentrated by evaporator, dissolved into ethyl acetate, to obtain 9.1 g (47.1 mmol) of 3-(butoxyethyl)aniline as a light brown oil at an yield of 89.8%.

**Synthetic Example 4** (Synthesis of an intermediate compound)

Synthesis of 3-[(1-butoxy-2-methyl)propyl]aniline [$R^1 = CH_3(CH_3)CH$, $R^2 = CH_3CH_2CH_2CH_2$, $X^1 = H$ in formula (II)]

a) Synthesis of 2-methyl-1-phenylpropanone
[$R^1 = CH_3(CH_3)CH$, $X^1 = H$ in formula (XV)]

250 ml of dichloromethane was charged into a 1 l reaction flask, followed by the addition of 67 g (502.5 mmol) of $AlCl_3$. After cooling the mixture on an ice water bath to 5 °C, 53.3 g (500.2 mmol) of isobutyryl chloride [$R^1 = i\text{-}C_3H_7$, $X^1 = X^2 = H$ in formula (XIV)] was added dropwise.

Then, 39.06 g (500 mmol) of benzene was added dropwise, while maintaining the temperature at 5 °C, and the mixture was reacted for 2 hours at 5 °C and 1 hour at 10 °C, followed by refluxing for 1 hour. The resulting reaction mixture was poured into ice water, washed with water, and dried to obtain a concentrated crude product. This crude product was distilled under vacuum to obtain 64.7 g (436.5 mmol) of 2-methyl-1-phenylpropanone as colorless oil at an yield of 87.3%.

b) Synthesis of 2-methyl-1-(3-nitrophenyl)propanone
[$R^1 = CH_3(CH_3)CH$, $X^1 = H$ in formula (XVI)]

150 ml of 95% sulfuric acid was charge into a 500 ml reaction flask and cooled to -20 °C in a dry ice-acetone bath. 15 ml of a concentrated nitric acid (61%, d = 1.38) was added dropwise in portions and the mixture was cooled to -20 °C. To this was added dropwise in portions 29.6 g (199.7 mmol) of 2-methyl-1-phenyl propanone so as not to raise the temperature above -5 °C. The mixture was reacted for 1 hour at -5 to -10 °C. After the reaction, the resulting reaction mixture was poured into ice water, extracted with ethyl acetate, washed with water, and dried to concentrate, thus obtaining 38.0 g (196.7 mmol) of a light brown oily product of 2-methyl-1-(3-nitrophenyl)propanone at an yield of 98.5%.

c) Synthesis of α-(1-methylethyl)-3-nitrobenzenemethanol
[$R^1 = CH_3(CH_3)CH$, $X^1 = H$ in formula (XVI)]

33 g (170.8 mmol) of 2-methyl-1-(3-nitrophenyl)-propanone was charged into a 500 ml eggplant type flask. 100 ml of ethanol was added and the mixture was cooled on an ice water bath. To this, 4.2 g (111 mmol) of $NaBH_4$ was slowly added and the mixture was reacted for 30 minutes under ice cooling and for 2 hours at room temperature. The resulting reaction liquid was concentrated by evaporator, and the residue was washed with water to quantitatively obtain α-(1-methylethyl)-3-nitrobenzenemethanol as a light brown oily product.

d) Synthesis of 1-[(1-butoxy-2-methyl)propyl]-3-nitrobenzene [$R^1 = CH_3(CH_3)CH$, $R^2 = CH_3CH_2CH_2CH_2$, $X^1 = H$ in formula (XIX)]

30 ml of n-hexane was charged in a 300 ml reaction flask, followed by the addition of 4.4 g (110 mmol) of 60% oily NaH. The mixture was thoroughly shaken to remove n-hexane by decantation. 30 ml of n-hexane was added to repeat the above procedure to remove oily component of NaH. 100 ml of DMF was added under ice cooling. To this was added 19.5 g (99.9 mmol) of α-(1-methylethyl)-3-nitrobenzene methanol [$R^1 = CH_3(CH_3)CH$, $X^1 = H$ in formula (XVI)] in portions to convert it into Na-oxide. 20.6 g (150.3 mmol) of 1-bromo-n-butane was added dropwise and reacted for 30 minutes under ice cooling and for 2

hours at room temperature. The resulting reaction mixture was poured into ice water and extracted with ethyl acetate to obtain 23 g of a crude product. This crude product was purified by column chromatography using a 5:1 mixture of n-hexane and ethyl acetate as a solvent to obtain 18.5 g (73.6 mmol) of light brown oily product of 1-[(1-butoxy-2-methyl)propyl]-3-nitrobenzene at an yield of 73.7%.

e) Synthesis of 3-[(1-butoxy-2-methyl)propyl]aniline
[$R^1 = CH_3(CH_3)CH$, $R^2 = CH_3CH_2CH_2CH_2$, $X^1 = H$ in formula (II)]

12.5 g (49.7 mmol) of 1-[(1-butoxy-2-methyl)propyl]-3-nitrobenzene, 60 ml of ethanol, 1.2 g of activated charcoal, 0.3 g of $FeCl_3 \bullet 6H_2O$, and 1 ml of hydrazine hydrate were charged into a 300 ml reaction flask. After refluxing for 15 minutes, 12 ml of hydrazine hydrate was added dropwise over 30 minutes, followed by refluxing for a further 3 hours. The reaction liquid was aspirated through a filter paper to remove activated charcoal, and the residue was concentrated by evaporator and dissolved into ethyl acetate, washed with water, and dried to concentrate to obtain 10 g a crude product. This crude product was purified by column chromatography using a 10:1 mixture of n-hexane and ethyl acetate as a solvent to obtain 8.5 g (38.4 mmol) of light yellow oily product of 3-[(1-butoxy-2-methyl)propyl]aniline at an yield of 77.3%.

**Formulation Example 1** 〈Dust〉

| | parts by weight |
|---|---|
| Compound 1 | 3 |
| Clay | 40 |
| Talc | 57 |

The above ingredients were pulverized and mixed to obtain a dust.

**Preparation Example 2** 〈Wettable Powder〉

| | parts by weight |
|---|---|
| Compound 3 | 50 |
| Lignin sulfonate | 5 |
| Alkyl sulfonate | 3 |
| Diatomaceous earth | 42 |

The above ingredients were pulverized and mixed to obtain wettable powder.

**Preparation Example 3** 〈Granules〉

| | parts by weight |
|---|---|
| Compound 4 | 5 |
| Bentonite | 43 |
| Clay | 45 |
| Lignin sulfonate | 7 |

The above ingredients were mixed and kneaded with the addition of water. The mixture was extruded and then dried to obtain granules.

**Preparation Example 4** ⟨Emulsion Concentrate⟩

| | parts by weight |
|---|---|
| Compound 12 | 20 |
| Polyoxyethylene alkylaryl ether | 10 |
| Polyoxyethylenesorbitan monolaurate | 3 |
| Xylene | 67 |

The above ingredients were mixed and dissolved to obtain an emulsion concentrate.

**Test Example 1**

⟨Test for controlling effect against cucumber gray mold (Botrytis cinerea on cucumber)⟩

A wettable powder similar to that prepared in Formulation Example 2, diluted with water to a prescribed concentration, was sprayed onto the cucumber plants of the first leaf stage (variety: *SAGAMI HAMPAKU*, one plant per pot) growing in an unglazed pot (diameter: 10 cm), in an amount of 5 ml per pot. After drying the sprayed leaves in the air, a circular cutting (diameter: 4 mm) of agar containing Botrytis cinerea, which had been cultured in advance in a potato-sucrose agar medium at 20 °C for 3 days, was attached directly to the center part of the leaves, and the pots maintained at 20-22 °C under high humidity conditions. Three days after the inoculation, lesion areas of the disease were examined to calculate the inhibition rate according to the following equation.

$$\text{Inhibition Rate (\%)} = \left[ 1 - \frac{\text{Lesion area in treated plot}}{\text{Lesion area in untreated plot}} \right] \times 100$$

The results are shown in Table 3.

**Test Example 2**

⟨Test for controlling effect against cucumber downy mildew (*Pseudoperonospora cubensis* on cucumber)⟩

A wettable powder similar to that prepared in Formulation Example 2, diluted with water to a prescribed concentration, was sprayed onto the cucumber plants of the second-leaf stage (variety: ***SAGAMI HAMUPAKU***, one plant per pot and 3 pots were used in the treated plot) growing in an unglazed pot (diameter: 10 cm), in an amount of 5 ml per pot. After drying the sprayed leaves in the air, a suspension of the spores of *Pseudoperonospora cubensis*, which had been collected from contracted leaf of cucumber, was sprayed onto the leaves, and the pots were maintained at 20-22 °C under high humidity conditions for 24 hours. 5-7 days after the inoculation, lesion areas of the disease were determined to calculate the inhibition rate according to the above equation.

The results are shown in Table 3.

**Test Example 3**

⟨Test for controlling effect against tomato late blight (*Phytophthora infestans* on tomato)⟩

A wettable powder similar to that prepared in Formulation Example 2, diluted with water to a prescribed concentration, was sprayed onto the young seedlings of tomato of the third leaf stage (variety: *FUKUJU* No. 2, one plant per pot and 3 pots were used in the treated plot) growing in an unglazed pot (diameter: 10 cm), in an amount of 5 ml per pot. After drying the sprayed leaves in the air, a suspension of the spores of *Phytophthora infestans*, which had been collected from contracted leaf of tomato, was sprayed onto the leaves, and the pots were maintained at 20-22 °C under high humidity conditions for 24 hours, followed by

growing in a greenhouse. 5-7 days after the inoculation, lesion areas of the disease were determined to calculate the inhibition rate according to the above equation.

The results are shown in Table 3.

**Test Example 4**

⟨Test for controlling effect against wheat red rust (*Puccinia recondita* on wheat)⟩

A wettable powder similar to that prepared in Formulation Example 2, diluted with water to a prescribed concentration, was sprayed onto the young seedlings of wheat of the second leaf stage (variety: *NORIN* No. 64, 16 seedlings per pot) growing in an unglazed pot (diameter: 10 cm), in an amount of 5 ml per pot. After drying the sprayed leaves in the air, a suspension of the uredospores of *Puccinia recondita*, which had been collected from contracted leaf of wheat, was sprayed onto the leaves, and the pots were maintained at 20-23 °C under high humidity conditions for 24 hours, followed by growing in a greenhouse. 7-10 days after the inoculation, lesion areas of the disease were determined to calculate the inhibition rate according to the above equation.

The results are shown in Table 3.

**Test Example 5**

⟨Test for controlling effect against wheat powdery mildew (*Erysiphe graminis f. sp. tritici* on wheat)⟩

A wettable powder similar to that prepared in Preparation Example 2, diluted with water to a prescribed concentration, was sprayed onto the young seedlings of wheat of the second leaf stage (variety: *NORIN* No. 64, 16 seedlings per pot) growing in an unglazed pot (diameter: 10 cm), in an amount of 5 ml per pot. After drying the sprayed leaves in the air, a suspension of the spores of *Erysiphe graminis f. sp. tritici*, which had been collected from the attacked leaf of wheat, was sprayed onto the leaves, and the pots were maintained at 20-24 °C under high humidity conditions for 24 hours, followed by growing in a greenhouse. 9-11 days after the inoculation, lesion areas of the disease were determined to calculate the inhibition rate according to the above equation.

The results are shown in Table 3.

TABLE 3

| Compound No. | Inhibition rate (%) at concentration of 500 ppm | | | | |
|---|---|---|---|---|---|
| | Cucumber gray mold | Cucumber downy mildew | Tomato late blight | Wheat leaf rust | Wheat powdery mildew |
| 1 | 90 | | | | |
| 2 | 90 | | | | |
| 3 | 90 | | | | |
| 4 | 90 | | | | |
| 5 | 80 | | | | |
| 6 | 88 | | | | |
| 7 | 95 | | | | |
| 8 | 86 | | | | |
| 9 | 60 | | | | |
| 10 | 80 | | | | |
| 11 | 72 | | | | |
| 12 | 84 | | | | |
| 13 | 90 | | | | |
| 14 | 88 | | | | |
| 15 | 90 | | | | |
| 16 | 94 | | | | |
| 17 | 66 | | | | |
| 18 | 93 | 68 | 63 | 55 | 57 |
| 19 | 94 | 49 | 75 | 57 | 67 |
| 20 | 67 | | | | |
| 21 | 94 | | | | |

Obviously, numerous modifications and variations of the present invention are possible in light of the above teachings. It is therefore to be understood that within the scope of the appended claims, the invention may be practiced otherwise than as specifically described herein.

## Claims

**1.** A derivative of 1-phenyl-5-heterocyclic-1H-1,2,4-triazole-3-carboxamide represented by the following formula (I),

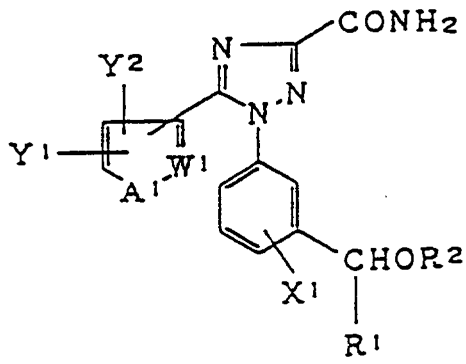

(I)

wherein $R^1$ is a $C_1$-$C_6$ alkyl group, $R^2$ is a $C_1$-$C_8$ alkyl group or a phenylmethyl group, $X^1$ represents a hydrogen atom, a $C_1$-$C_4$ alkyl group or a halogen atom, $Y^1$ is a hydrogen atom or a $C_1$-$C_4$ alkyl group, $Y^2$ is a hydrogen atom, a $C_1$-$C_4$ alkyl group, or a halogen atom, $A^1$ represents a group -N=CH-, -NH-, -N($C_1$-$C_4$ alkyl)-,-N(Ph)-, -O-, or -S-, and $W^1$ denotes a group -C($W^2$)= or -N=, wherein $W^2$ is a hydrogen atom, a $C_1$-$C_4$ alkyl group, or a halogen atom.

**2.** 1-[3-[(1-Butoxy-2-methyl)propyl)phenyl]-5-(6-methylpyridin-2-yl)-1H-1,2,4-triazole-3-carboxamide.

**3.** 1-[3-[(1-Butoxy-2-methyl)propyl]phenyl]-5-(3-methylthiophen-2-yl)-1H-1,2,4-triazole-3-carboxamide.

**4.** A process for manufacturing derivative of 1-phenyl-5-heterocyclic-1H-1,2,4-triazole-3-carboxamide (I), which comprises reacting oxazoledione hydrazone derivative (VI) and ammonia to produce glycinamide derivative (VII) and subjecting the glycinamide derivative (VII) to a cyclocondensation reaction according to the following reaction formula (I),

**Reaction (I)**

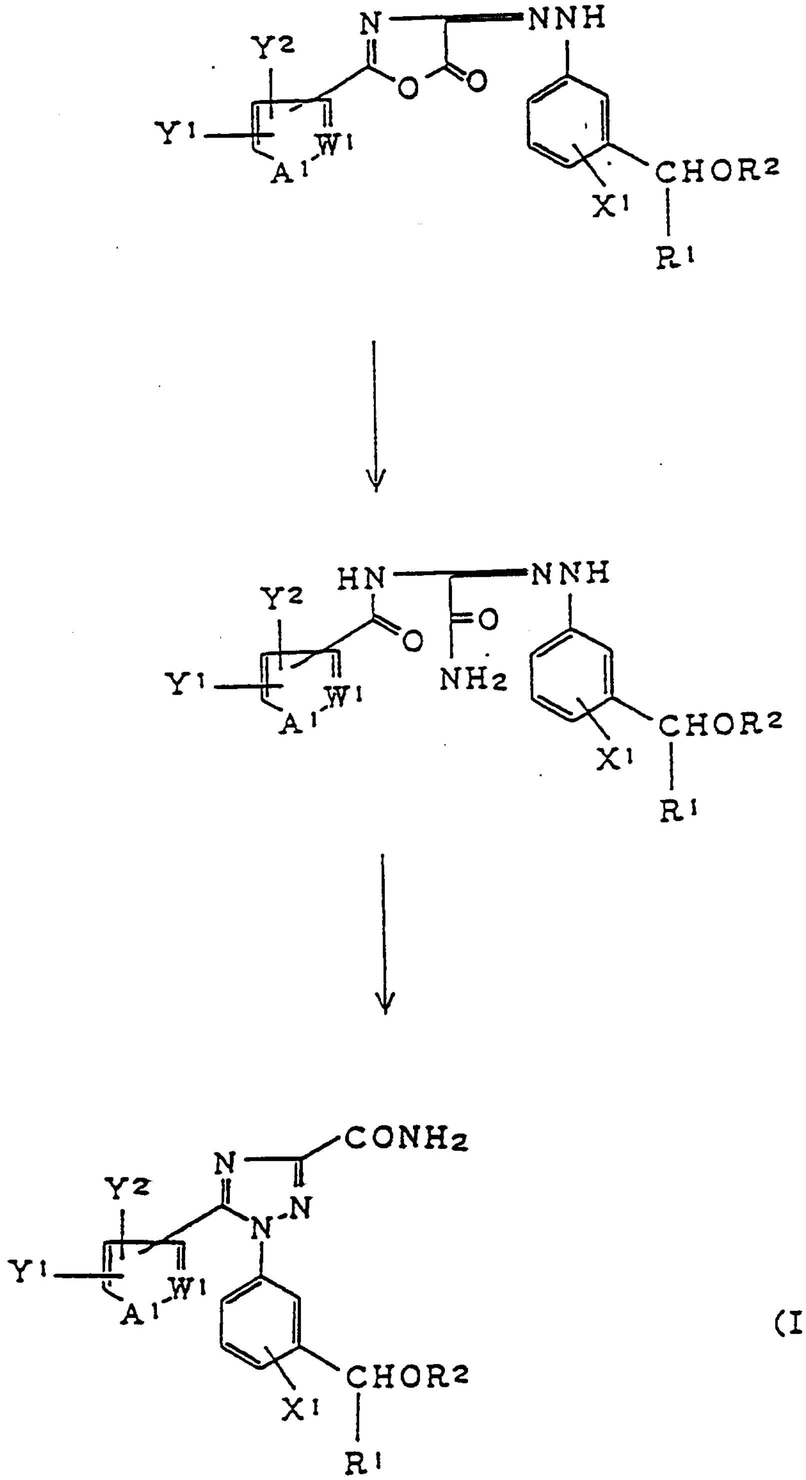

(VI)

(VII)

(I)

wherein $R^1$ is a $C_1$-$C_6$ alkyl group, $R^2$ is a $C_1$-$C_8$ alkyl group or a phenylmethyl group, $X^1$ represents a hydrogen atom, a $C_1$-$C_4$ alkyl group or a halogen atom, $Y^1$ is a hydrogen atom or a $C_1$-$C_4$ alkyl group, $Y^2$ is a hydrogen atom, a $C_1$-$C_4$ alkyl group, or a halogen atom, $A^1$ represents a group -N=CH-, -NH-, -N($C_1$-$C_4$ alkyl)-,-N(Ph)-, -O-, or -S-, and $W^1$ denotes a group -C($W^2$)= or -N=, wherein $W^2$ is a hydrogen atom, a $C_1$-$C_4$ alkyl group, or a halogen atom.

5. A process for manufacturing a derivative of 1-phenyl-5-heterocyclic-1H-1,2,4-triazole-3-carboxamide (I), which comprises reacting oxamide derivative (X) and aldehyde derivative (XI) to produce dihydrotriazole carboxamide (XII) and oxidizing the dihydrotriazole carboxamide (XII) according to the following reaction formula (II),

**Reaction (II)**

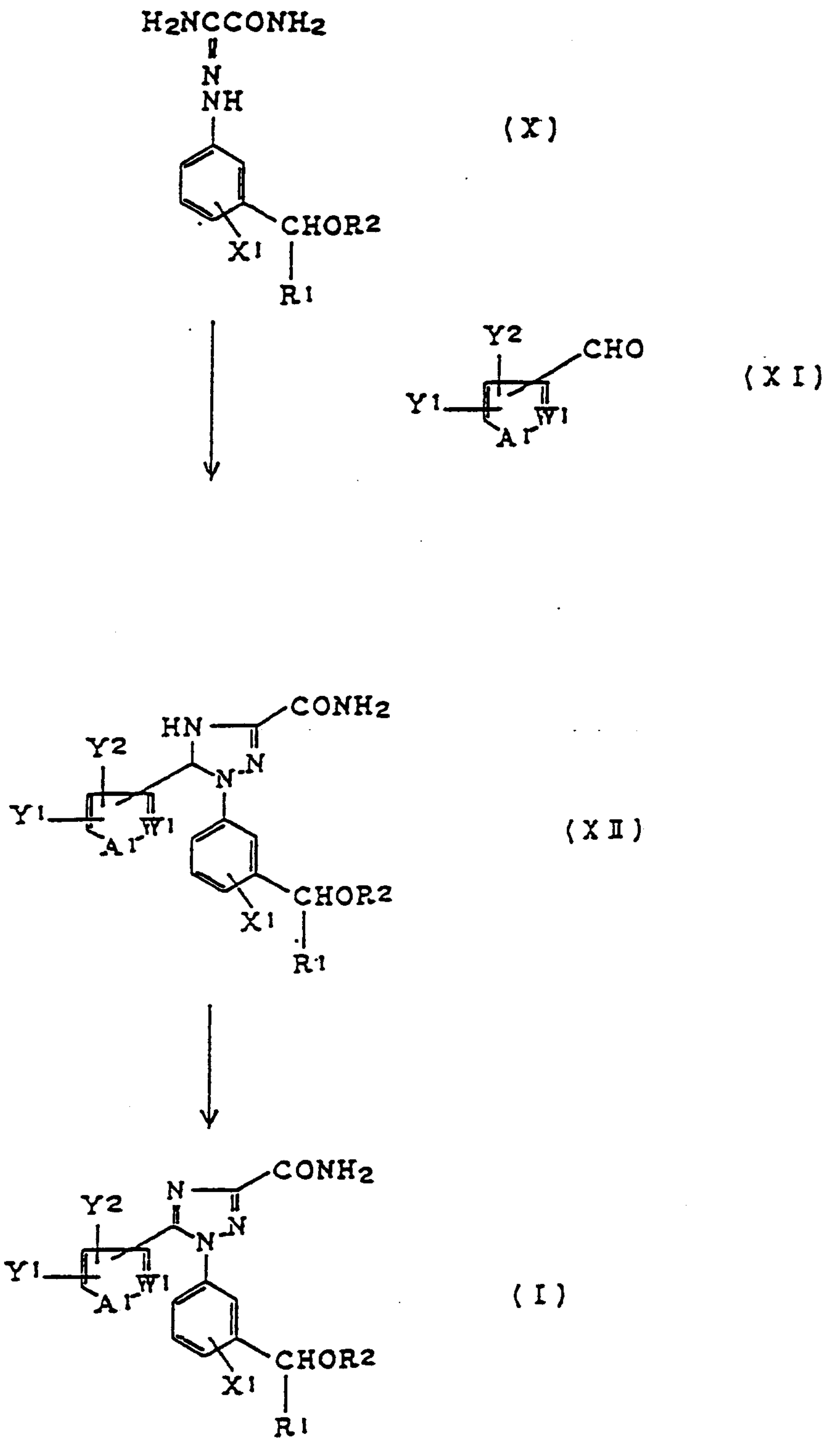

(X)

(XI)

(XII)

(I)

wherein $R^1$ is a $C_1$-$C_6$ alkyl group, $R^2$ is a $C_1$-$C_8$ alkyl group or a phenylmethyl group, $X^1$ represents a hydrogen atom, a $C_1$-$C_4$ alkyl group or a halogen atom, $Y^1$ is a hydrogen atom or a $C_1$-$C_4$ alkyl group,

$Y^2$ is a hydrogen atom, a $C_1$-$C_4$ alkyl group, or a halogen atom, $A^1$ represents a group -N=CH-, -NH-, -N($C_1$-$C_4$ alkyl)-,-N(Ph)-, -O-, or -S-, and $W^1$ denotes a group -C($W^2$)= or -N=, wherein $W^2$ is a hydrogen atom, a $C_1$-$C_4$ alkyl group, or a halogen atom.

**6.** A fungicidal composition comprising the dervative of 1-phenyl-5-heterocyclic-1H-1,2,4-triazole-3-carboxamide (I) defined in Claim 1 as an active ingredient.

**7.** A fungicidal composition for agriculture and horticulture, comprising the dervative of 1-phenyl-5-heterocyclic-1H-1,2,4-triazole-3-carboxamide (I) defined in Claim 1 and a carrier.

**8.** A fungicidal composition according to Claim 7, which is effective for cucumber gray mold.

European Patent Office

# EUROPEAN SEARCH REPORT

Application Number

EP 93 10 4527

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 282 303 (KUREHA CHEMICAL INDUSTRY CO., LTD.; JAPAN (JP)) 14 September 1988 * the whole document * | 1-8 | C07D401/04 C07D409/04 A01N43/653 |
| D,A | EP-A-0 220 956 (KUREHA CHEMICAL INDUSTRY CO., LTD.; JAPAN (JP)) 6 May 1987 * the whole document * | 1-8 | |
| D,A | EP-A-0 282 669 (KUREHA CHEMICAL INDUSTRY CO., LTD.; JAPAN (JP)) 21 September 1988 * the whole document * | 1-8 | |
| A | EP-A-0 217 552 (SUMITOMO CHEMICAL CO.) 8 April 1987 * the whole document * | 1-8 | |

TECHNICAL FIELDS SEARCHED (Int. Cl.5)

C07D

The present search report has been drawn up for all claims

| Place of search | Date of completion of the search | Examiner |
|---|---|---|
| THE HAGUE | 15 JUNE 1993 | Bernd Kissler |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document